# EUROPEAN PATENT APPLICATION

(11) **EP 3 563 871 A1**
(43) Date of publication of application: **06.11.2019**
(21) Application number: 18170186.3
(22) Date of filing: 30.04.2018
(51) Int. Cl.: A61K 47/00, A61K 8/00, A61L 24/00, C08J 3/075, C08J 3/24

(54) **SELF-HEALING CROSS-LINKABLE SHELLS**

(71) Applicant: ECOLE POLYTECHNIQUE FEDERALE DE LAUSANNE (EPFL), 1015 Lausanne (CH)
(72) Inventor: The designation of the inventor has not yet been filed
(74) Representative: Gurney, Steven

(57) **Abstract**

The present invention relates to self-healing shells comprising a self-healing shell incorporating amphiphilic molecules having metal-coordinating group(s) reversibly cross-linked with metal cations.

## Description

### Field of the Invention

The present invention relates to self-healing shells comprising amphiphilic molecules having metal-coordinating group or groups reversibly cross-linked with suitable metal cations, their uses and processes to produce them.

### Background to the Invention

Surfactants (or surface active agents) are amphiphilic molecules comprising both a hydrophobic and a hydrophilic group. The surfactant therefore contains both a water soluble portion and a water insoluble (oil soluble) portion so that a surfactant will self-assemble at a water or gas and oil interface, with the hydrophilic group extending into the water phase and the hydrophobic group extending into the oil or gas phase.

Surfactants have many uses, including as detergents, wetting agents, emulsifiers, foaming agents, and dispersants.

However, there remains a need to develop surfactant systems containing enhanced properties.

### Summary of the Invention

In a first aspect of the present invention there is provided a self-healing shell at an interface between a first fluid phase and a second fluid phase; wherein the first fluid phase is a liquid phase or a gas phase and the second fluid phase is a liquid phase; said shell comprising amphiphilic molecules and metal cations; wherein the amphiphilic molecules comprise one or more hydrophilic group(s) and one or more hydrophobic group(s), and wherein the amphiphilic molecules comprise one or more metal-coordinating group(s); wherein the metal cations comprise metal ions, metal oxides, metal hydroxides, metal carbides, metal nitrides, and/or metal nanoparticles; and wherein amphiphilic molecules in the shell are reversibly cross-linked via the one or more metal-coordinating group(s) and metal cations.

It has surprisingly been discovered that by using amphiphilic molecules with a metal coordinating group, it is possible to combine the interfacial activity of surfactants with the reversible gelling and/or self-healing properties of the meal coordinating groups. This allows the surfactants to reversibly cross-link (self-heal) only at interfaces, thereby forming shells that display self-healing properties.

In a yet further aspect of the present invention, there is provided a method of manufacturing self-healing shells as defined herein; the method comprising forming a system comprising: an interface between a first fluid phase and a second fluid phase, amphiphilic molecules and metal cations; wherein the first fluid phase is a liquid phase or a gas phase and the second fluid phase is a liquid phase; such that the metal-coordinating group(s) and metal cation(s) reversibly cross-link at the interface to form a self-healing shell.

In a yet further aspect of the present invention, there is provided a method of transporting material from a first closed system to a second closed system within a surrounding solvent using self-healing shells described herein; comprising forming a first self-healing capsule comprising a shell to encapsulate a first fluid and form a first closed system; forming a second self-healing capsule comprising shell to encapsulate a second fluid and form a second closed system; bringing the first and second self-healing capsules into contact such that the first and second fluids fuse bringing the first and second closed systems into contact with each other while still being protected from the surrounding solvent by the self-healing shell.

In a yet further embodiment of the present invention, there is provided the use of a self-healing shell as described herein in a screening assay. Screening assays which use self-healing shells according to the present invention may to reduce cross-talk between droplets caused by material leakage. The present invention can be used to stabilise emulsion drops used in screening assays.

In a yet further embodiment of the present invention, there is provided the use of self-healing shell as described herein as a vehicle for drug delivery and/or for encapsulation of drugs, food ingredients, nutraceuticals, cosmetics, pesticides, nutrients, fragrances, catalysts, agrichemicals, reagents to form proteins, coatings, paints, or waste products.

In a yet further embodiment of the present invention, there is provided three-dimensional hydrogel material comprising self-healing shells as described herein.

In a yet further embodiment of the present invention, there is provided an amphiphilic molecule as described herein, comprising one or more hydrophilic group(s) and one or more hydrophobic group(s); and wherein the amphiphilic molecule comprises one or more metal-coordinating group(s) as claimed in; wherein the amphiphilic molecule is a block copolymer; and wherein the metal-coordinating group is a metal-coordinating group selected from the group consisting of: benzenediol or derivatives thereof, preferably catechol or derivatives thereof; and benzenetriol or derivatives thereof, preferably gallol or derivatives thereof; histidines and derivatives thereof; ethylenediaminetetraacetic acid and derivatives thereof and wherein the metal-coordinating group may optionally be further substituted.

### Description of the Drawings

Figure 1 shows examples of metal coordinating groups coordinated to a metal cation.
Figure 2 shows microscope images of oil and surfactant (1 wt.% FSHDopa) in water drop during removal or injection of liquid
Figure 3 shows microscope images of oil and surfactant drops (HFE 7100 + 1 wt.% FSHDopa) being brought into contact with each other
Figure 4 is shows microscope images of oil and surfactant drops (HFE7100 + 1 wt.% FSHDopa) showing the transportation of contents from one drop to another
Figure 5 shows water in oil in water double emulsions with the oil layer containing FSHPEG900HA surfactant cross-linked with iron ions, demonstrating reduced leakage of fluorescent dye
Figure 6 shows (a) 1 wt.% FSHDopa in HFE7100 containing FeCl₃ and (b) catechol in HFE7100 containing FeCl₃
Figure 7 shows the interfacial tension between 1 wt.% FSHDopa in HFE 7500 and water that has a pH of 3.5 (red shaded area) and water that has a pH of 12 (green shaded area)
Figure 8 shows HFE 7100 drop containing 1 wt%. FSHDopa and iron ions in water
Figure 9 shows time lapse micrographs of a drop composed of ethyl acetate containing 0.1 wt.% SA-Dopa that is formed in a continuous phase composed of water at pH=7.7
Figure 10 shows time lapse optical micrographs of a drop composed of toluene containing 1wt.% DiDopa-PPG and iron ions that is formed in water at pH = 7.7
Figure 11 shows fluorescent intensity of cores of water-oil-water double emulsions as a function of time for double emulsions stabilized with a non-crosslinkable surfactant (DiFSHPEG900) (blue) and those stabilized with crosslinkable FSHPEG900HA surfactant (red)

### Detailed Description of the Invention

The following embodiments apply to all aspects of the present invention.

In the present application, a number of general terms and phrases are used, which should be interpreted as follows.

"Self-healing" means the shell is able to spontaneously repair if it is breached. The shell is able to reform cross-links either with the shell and/or via further surfactant molecules and cations within the liquid(s) to reform an intact shell. The repair may reform the shell to its original structure or it may reform the shell in a slightly different structure that nevertheless repairs the breach. The property of self-healing can make the shell "sticky".

"Shell" means the amphiphilic molecules at the interface are ionically cross-linked via the metal coordinating group and the metal cation to form a responsive shell which separates the first phase and the second phase. The shell may encapsulate the first phase from the second phase. The shell may form a capsule. The shell may form a membrane between the first phase and second phase. The membrane may be planar or another shape. There may be multiple phases beyond the first and second phase.

The shell protects the inner phase from the outer phase (and vice versa) and provides a resilient but flexible shell. The shell is stable and strong enough to be moved within a system and will self-heal if it is breached by the reformation of further ionic coordination bonds.

"Capsule" according to the present invention means an inner phase encapsulated by a shell that is formed at the interface between the first phase and the second phase. For example, in an oil-in-water emulsion, drops of oil may be encapsulated by a shell according to the present invention thereby protecting the inner oil from the outer water phase. Equally, in a water-in-oil emulsion, drops of water may be encapsulated by a capsule according to the present invention to protect the inner water phase from an outer oil phase. Water-in-oil-in-water and oil-in-water-in-oil emulsions or emulsion drops encompassing more than one smaller drop are also possible. Further, it is possible to form capsules according to the present invention when the first and second phases have some solubility in each other, provided the interfacial tension is sufficiently high for drops to form. If mechanical agitation is used to form drops, the interfacial tension between two liquids can be quite small.

Once capsules have been formed, they can be transferred (or the medium surrounding them can be replaced). This could include removing them from the system into air or moving them into any suitable medium. It is therefore possible in embodiments to remove formed capsules and move them into a different system, or to form capsules and then change the system in which they are placed. The shell of the present invention protects the encapsulated contents from the medium and vice versa. As an example, formed capsules encapsulating a liquid could be introduced into the same liquid contained in the core of the capsule, but the inner liquid is still separated since it is encapsulated by the shell.

"Membrane" according to the present invention means that the shell forms at the interfacial boundary. This may be planar or conform to a different shape. An example of a membrane is where a first phase is introduced into a second phase via, for example, a needle. A shell will form at the interfacial boundary but will not fully encapsulate the inner material since it is still attached to the needle. In this example, ultimately, if the drop releases from the needle the shell may fully encapsulate the inner material forming a capsule.

"Amphiphilic molecule": the shell according to the present invention is formed from amphiphilic molecules coordinated via metal cations which undergo molecular self-assembly at interfaces to form self-healing shells. By molecular self-assembly it is intended to mean that the amphiphilic molecules form a suitable structure to enable a shell to form. Examples of suitable structures include monolayers, bilayers or aggregates.

Amphiphilic molecules comprise: a hydrophilic group and a hydrophobic group. The group may be a group, tail or block. Thus, any suitable amphiphilic molecules are encompassed by the present invention. The amphiphilic molecule may comprise a hydrophilic head, a hydrophilic tail or include a hydrophilic block polymer. The amphiphilic molecule may comprise a hydrophobic head, a hydrophobic tail or include a hydrophobic block polymer.

In a preferred embodiment, the amphiphilic molecule may be a block copolymer incorporating at least one hydrophobic and hydrophilic block. The amphiphilic molecule may be a diblock copolymer. The amphiphilic molecule may be a triblock copolymer.

The amphiphilic molecules also comprise one or more metal coordinating group(s). Incorporating metal coordinating group or groups into the amphiphilic molecules enables the combination of interfacial activity of surfactants with the reversible gelling and self-healing properties of the metal coordinating groups. These groups allow the surfactants to reversibly cross-link (self-heal) at interfaces, thereby converting the surfactants into shells that display self-healing properties. It is at the interface that the surfactants will self-assemble and begin to cross-link which is where the shell will begin to form. The shell may then grow through the attachment of additional surfactants at or near the interface.

Under suitable conditions, the amphiphilic molecules according to the present invention self-assemble and reversibly cross-link with suitable metal cations into shells. This may be pH dependent, and the present invention therefore provides for the formation of materials which are pH responsive. The shells may also be tunable depending on the cation introduced into the system.

The pH dependent nature of the molecules means that it is possible to include the reagents into a system and, upon shifting the pH to a suitable value (for example a suitable basic pH), the molecules will cross-link to form the shell.

"Hydrophilic group" means a polar component which is soluble in water or other polar solvents. Suitable hydrophilic groups include hydrophilic polymers, or hydrophilic block polymers. Suitable hydrophilic groups include polyethyleneglycol (PEG) polyacrylicacid (PAA), polyethyleneimine (PEI), polyvinylalcohol (PVA), Poly(N-isopropylacrylamide) (PNIPAM), poly(2-methyl-2-oxazoline) (PMOXA), polyglycols, natural hydrophilic polysaccharides including dextran, alginate, and peptides.

The amphiphilic molecule may include two or more hydrophilic groups. The metal coordinating group may itself constitute the or a hydrophilic group. As an example, the amphiphilic molecule may comprise a hydrophilic block polymer and a hydrophilic metal coordinating group (e.g catechol or derivatives thereof).

"Hydrophobic group" means a non-polar group which is a water-insoluble (or oil soluble) component. Suitable hydrophobic groups include substituted or unsubstituted lipophilic hydrocarbon chains, fluorinated chains, hydrophobic polymer(s), liquid crystals and the like.

Lipophilic hydrocarbon chains are preferably branched or unbranched having from 6 to 18 carbon atoms. The carbon chain may be saturated or unsaturated. The carbon chains may optionally be substituted with one or more functional groups and may contain one or more heteroatoms. Suitable lipophilic hydrocarbon chains include short saturated or unsaturated aliphatic chains (for example the hydrophobic part of lipids).

Hydrophobic polymers include: aliphatic chains (saturated and unsaturated), acrylics, amides and imides, carbonates, dienes, esters, ethers, fluorocarbons, perfluorinated polyethers, olefins, styrenes, vinyl acetals, vinyl and vinylidene chlorides, vinyl esters, vinyl ethers and ketones, vinylpyridine and vinypyrrolidone polymers. Hydrophobic polymers may preferably be selected from polypropyleneglycol (PPG), polystyrene (PS), polylacticacid (PLA), fluorocarbons, methacrylates, polyethylene, polydimethylsiloxane (PDMS), chitosan and cellulose.

Hydrophobic polymer(s) are particularly preferably fluorinated. Preferred fluorinated polymers include perfluoropolyether groups. Particular polymers include perfluorinated polyethers with carboxylic acid-, methyl ester-, methylene alcohol- or allyl ether end groups. Examples of suitable perfluoropolyether puorinated polymers include Krytox FSH 157, Krytox FSM 157, Krytox FSL 157, FC40.

Fluorinated polymers may comprise any fluorinated compound such as a linear, branched, cyclic, saturated, or unsaturated fluorinated hydrocarbon. The fluorinated molecule can optionally include at least one heteroatom. In some cases, the fluorophilic compound may be highly fluorinated, for example at least 30%, at least 50%, at least 70%, or at least 90% of the hydrogen atoms are replaced by fluorine atoms. In a further embodiment, all of the hydrogen atoms on the fluorinated part of the molecule are replaced by fluorine atoms.

Fluorinated polymers may include one or more fluorinated compounds selected from: perfluorodecalin, perfluoromethyldecalin, perfluoroindane, perfluorotrimethyl bicyclo[3.3.1]nonane, perfluoromethyl adamantine, perfluoro-2,2,4,4-tetra-methylpentane; 9-12C perfluoro amines, e.g., perfluorotripropyl amine, perfluorotributyl amine, perfluoro-1-azatricyclic amines; bromofluorocarbon compounds, e.g., perfluorooctyl bromide and perfluorooctyl dibromide; F-4-methyl octahydroquinolidizine and perfluoro ethers, including chlorinated polyfluorocyclic ethers, perfluoro-4-methylmorpholine, perfluorotriethylamine, perfluoro-2-ethyltetrahydrofuran, perfluoro-2-butyltetrahydrofuran, perfluoropentane, perfluoro(2-methylpentane), perfluorohexane, perfluoro-4-isopropylmorpholine, perfluorodibutyl ether, perfluoroheptane, perfluorooctane, perfluorotripropylamine, perfluorononane, perfluorotributylamine, perfluorodihexyl ether, perfluoro[2-(diethylamino)ethyl-2-(N-morpholino)ethyl]ether, n-perfluorotetradecahydrophenanthrene, and mixtures thereof. In some instances, the fluorophilic component can be straight-chained, branched, cyclic, etc., and/or have a combination of such structures.

The amphiphilic molecule may be a block copolymer. Suitable block copolymers include: diblock copolymers, triblock copolymers, and/or random block copolymers. Suitable diblock copolymers include: AB diblock copolymers, PEG diblock copolymers, polystyrene diblock copolymers. Examples of suitable diblock polymers include such as PEG-poly(propylene glycol), PNIPAM-poly(propylene glycol), PMOXA-poly(propylene glycol), PEG-poly(lactic acid), PEG-poly(lactic-co-glycolic acid) or any combination of said blocks. Suitable triblock copolymers include: ABA triblock copolymers, ABC triblock copolymers, biodegradable triblock copolymers, PEG/PPG triblock copolymers, polystyrene triblock copolymers, multi-arm PEG block copolymers, dendrimer-based block-copolymers; PNIPAM-based block copoylmers, PIMOXA based block-copolymers, light responsive block copolymers, temperature responsive block copolymers, PPG, perfluorinated polyether, perfluorinated polyether-PEG. Suitable random block copolymers include any combination of the blocks outlined above, provided at least one block is hydrophobic and at least one block is hydrophilic. Particular random block polymers include perfluorinatedpolyether and polypropyleneglycol.

The amphiphilic molecule may be a bipolar amphiphilic molecule.

"Metal coordinating group" means a group which is able to coordinate with a metal cation by forming a reversible ionic bond between the coordinating group and the cation.

The metal coordinating group may be connected to the amphiphilic molecule, either directly or via a spacer. The metal coordinating group may itself form the (or a) hydrophilic group. A metal coordinating group may be located at or near the hydrophilic region(s) of the amphiphilic molecule. A metal coordinating group may be located at or near the hydrophobic region(s) of the amphiphilic molecule. Metal coordinating groups may be located at or near both the hydrophilic and hydrophobic region(s) of the amphiphilic molecule.

The spacer or linking group, if used, may be a small molecule chosen to link the groups together. Suitable linkers include, for example, PEG, aliphatic chains, short hydrocarbon-based chains, morpholino groups and/or a phosphate groups. The linker may be selected to assist with self-assembly and/or coordination.

The number of metal coordinating groups present on the amphiphilic molecule will vary in number and location depending on the desired properties. There could be one, two, three or more metal coordinating groups. If the molecule includes repeating units, the metal coordinating group could be present in the repeating portion thereby leading to a number of groups per molecule. In an embodiment, there is one metal coordinating group per amphiphilic molecule. In a further embodiment, there are two metal coordinating groups per amphiphilic molecule. In a yet further embodiment, there are three or more metal coordinating groups per amphiphilic molecule. In a further embodiment, the metal coordinating group is incorporated into the repeating unit of a polymer.

The ratio of metal coordinating group(s) to metal ions can be tuned. There may be one, two or three coordinating groups per metal ion. Preferred metal coordinating groups are benzenediol or derivatives thereof. Further preferred metal coordinating groups are benzenetriol or derivatives thereof. Further metal coordinating groups might be histidines or derivatives thereof, groups comprising a carboxyl group; and ethylenediaminetetraacetic acid and derivatives thereof. Preferred metal coordinating groups are benzenediol or benzenetriol. Particularly preferred metal coordinating groups are benzenediol or derivatives thereof.

"Benzenediol" means a benzene ring substituted with two hydroxyl groups and "Benzenetriol" means a benzene ring substituted with three hydroxyl groups. The benzene ring may optionally be further substituted. To provide sufficient complexation, the hydroxyl groups are adjacent to each other, e.g.in a benzenediol the ortho (catechol) isomer. Thus, in a preferred embodiment, the metal coordinating group is catechol (also known as 1,2-benzenediol) or a derivate thereof. For a benzenetriol, a preferred molecule is gallol.

In a preferred embodiment, two hydroxyl groups are in the ortho-meta positions vs the hydrophobic chain. In an alternative embodiment, two catechol hydroxyl groups are in the meta-para positions vs the hydrophobic chain. The meta-para position is especially preferred.

By using a benzenediol or benzenetriol as metal coordinating group it is not necessary to have a separate hydrophilic group. That is, the metal coordinating group functions as the hydrophilic group in the amphiphilic molecule. However, it may be desirable to nevertheless still have separate hydrophilic group or groups, for example a hydrophilic block polymer.

Further metal coordinating groups include specific catechols (such as dopamine, hydrocaffeic acid, and tiron (disodium 4,5-dihydroxy-1,3-benzenedisulfonate).

Yet further metal coordinating groups include amino acids. Suitable amino acids include histidine, serine, threonine, asparagine, glutamine, lysine, or cysteine.

"Metal cation" can be any metal cation suitable to coordinate with a metal coordinating group. The metal cation forms reversible ionic bonds with metal coordinating group(s). Suitable metal cations include metal ions, metal oxides, metal hydroxides, metal carbides, metal nitrides and/or metal nanoparticles.

Particular metal ions include beryllium, magnesium, calcium, strontium, barium, chromium, manganese, iron, cobalt, nickel, copper, silver, gold, zinc, cadmium, mercury, aluminium, gallium, indium, tin, lead and bismuth. Particularly preferred metal cations include iron, aluminium or titanium, with iron especially preferred.

More particularly, suitable cations include Be²⁺ beryllium ion, Mg²⁺ magnesium ion, Ca²⁺ calcium ion, Sr²⁺ strontium ion, Ba²⁺ barium ion, Ti²⁺ titanium (II), Ti⁴⁺ titanium (IV), Cr²⁺ chromium (II), Cr³⁺ chromium (III), Cr⁶⁺ chromium (VI), Mn²⁺ manganese (II), Mn³⁺ manganese (III), Mn⁴⁺ manganese (IV), Fe²⁺ iron (II), Fe³⁺ iron (III), Co²⁺ cobalt (II), Co³⁺ cobalt (III), Ni²⁺ nickel (II), Ni³⁺ nickel (III), Cu⁺ copper (I), Cu²⁺ copper (II), Ag⁺ silver ion, Au⁺ gold (I), Au⁺³ gold (III), Zn²⁺ zinc ion, Cd²⁺ cadmium ion, Hg₂²⁺ mercury (I), Hg²⁺ mercury (II), Al³⁺ aluminium ion, Ga³⁺ gallium ion, In⁺ indium (I), In³⁺ indium (III), Sn²⁺ tin (II), Sn⁴⁺ tin (IV), Pb²⁺ lead (II), Pb⁴⁺ lead (IV), Bi³⁺ bismuth (III), and Bi⁵⁺ bismuth (V). Particularly preferred metal cations include Fe³⁺ iron (III).

The metal may be added in the form of a metal salt. Suitable metal salts include but are not limited to halides, nitriles, hydroxides and the like.

The metal cation may be in the form of an oxide or nanoparticle. For example, iron oxide nanoparticles may be used. Other suitable oxides or nanoparticles include iron oxides, iron nitrides, iron carbides, nickel oxides, nickel carbides, titanium oxides, titanium metal particles, titanium nitrides, titanium carbides.

Using nanoparticles allows for larger numbers of metal coordinating groups to ionically bond with a single nanoparticle. This impacts the properties of the shell, namely increasing the relaxation time, and may increase the stability of the shell.

"Metal complexed" or "metal coordinated" means amphiphilic molecules coordinate via their metal coordinating groups and metal cations through ionic bonds. Through this coordination, the amphiphilic molecule form self-assembling, reversibly cross-linked shells that can self-heal via further reversible cross-linking.

"Viscoelastic" means the property of a substance of exhibiting both elastic and viscous behaviour, the application of stress causing temporary deformation if the stress is quickly removed but permanent deformation if it is maintained. The shells of the present invention may be viscoelastic.

The present invention provides for the first time a self-healing shell at an interface between a first fluid phase and a second fluid phase; wherein the first fluid phase is a liquid phase or a gas phase and the second fluid phase is a liquid phase; said shell comprising amphiphilic molecules and metal cations; wherein the amphiphilic molecules comprise one or more hydrophilic group(s) and one or more hydrophobic group(s), and wherein the amphiphilic molecules comprise one or more metal-coordinating group(s); wherein the metal cations comprise metal ions, metal oxides, metal hydroxides, metal carbides, metal nitrides and/or metal nanoparticles; and wherein amphiphilic molecules in the shell are reversibly cross-linked via the one or more metal-coordinating group(s) and metal cations.

It has surprisingly been discovered that by having metal-coordinating groups on amphiphilic molecules, these molecules can ionically cross-link at the interface with metal cations to form self-healing shells. These shells have a number of advantageous properties. The shells are self-healing meaning that in the event of rupture; new coordinations will occur reforming the intact shell. The shells are robust and will self-heal in the event of a rupture. In addition, the self-healing properties mean that a shell (for example in the form of a capsule) can be brought into contact with an adjacent shell (for example an adjacent capsule) and the shells can be fused. In the case of capsules, this enables the capsules to merge.

Furthermore, the mechanical properties of the shell can be adjusted depending on the choice of: metal-coordinating group; metal cation; the ratio of metal cation to metal coordinating group; and/or by varying other conditions like pH and temperature. The mechanical properties can also be modified by the number of metal coordinating groups attached to the amphiphilic molecule together with the location of their placement on the molecule. Properties can also be adjusted by the length and flexibility of spacers connecting metal coordinating groups to the surfactants. Properties can also be adjusted by the selection of hydrophobic and hydrophilic groups.

The robust self-healing nature of the shells together with their tuneable mechanical properties make the present invention useful in a variety of areas including transportation, storage or movement of target materials, for example drugs, foodstuffs, agrochemicals, cosmetics and the like.

Any suitable metal-coordinating group can be chosen. The metal-coordinating group may be the hydrophilic group on the amphiphilic molecule or may be otherwise attached to the amphiphilic molecule. The amphiphilic molecule may comprise one, two, three or more metal-coordinating groups. The groups may be at the end or ends of the molecule or may be in a repeating unit of a polymer. The amphiphilic molecule may be a bipolar amphiphilic molecule. Such molecules may assemble into a U shape at an interface, impacting their packing density at the interface.

One preferred metal-coordinating group is benzenediol or derivative thereof. The dual hydroxy groups on the molecule enable coordination with metal ions. In a preferred embodiment, it has been found that preferably catechol or derivatives thereof are particularly useful. Catechols having the two hydroxyl groups in the ortho and para positions relative to the hydrophobic chain are especially preferred. The benzenediol may be gallol having three adjacent hydroxy groups.

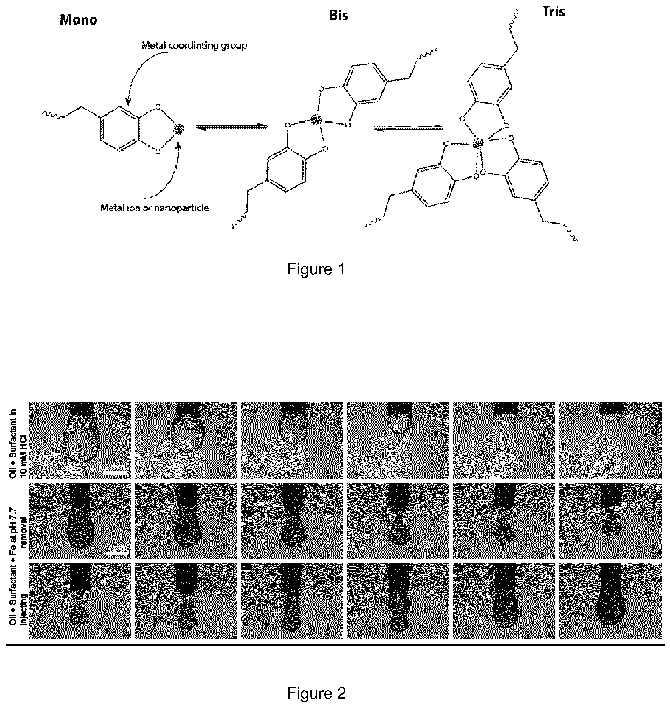

Catechols form reversible ionic bonds with metal cations. An example is shown in Figure 1 with metal ions. The catechol and metal ion can form a mono, bis or tris coordination and this interaction is pH dependent and dependent on the oxidation state of the metal ion. This allows the mechanical properties of the resultant shell to be tuned depending on the choice of metal ion and/or pH.

Alternatively, if the metal ion is, for example, a nanoparticle like iron oxide nanoparticles, then many more catechols can bind to a single nanoparticle. Such an approach may improve the stability of the shell, and may do so by shifting the relaxation time of the resultant structure.

Any suitable hydrophobic chain may be selected and the properties of the chain can be tuned to suit the needs of the self-healing shell.

In a particular embodiment, amphiphilic molecules include the following compounds of Formula I (a-e):

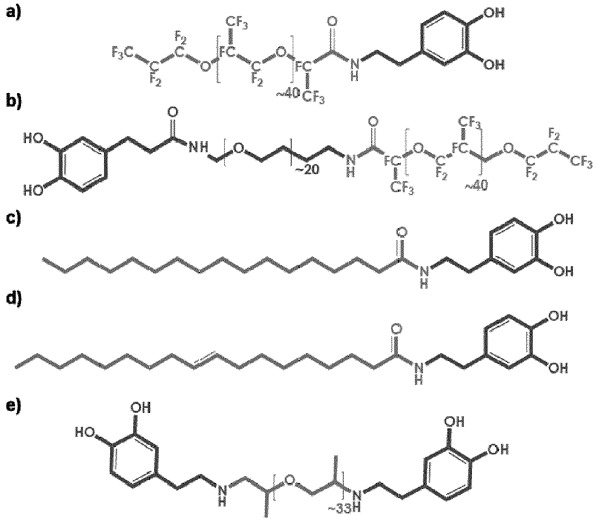

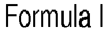

Formula I (a), FSHDopa, was synthesized by linking Dopamine to Krytox 157 FSH a perfluorinated polyether with a carboxylic acid end group. This synthesis is a two step synthesis. Formula I (b), FSHPEG900HA, to increase the size of the hydrophilic part, a PEG-spacer was added that separates the fluorinated block from the catechol. Formula I (c) stearic acid-dopa. Formula I (d), Oleic acid-dopa, the unsaturated bond in the hydrobarbon chain changes the stiffness of the molecule and therefore its packing density and propensity to form aggregates. Formula I (e), DiDopa-PPG, this surfactant contains two catechols that are separated by polypropylene glycol.

As discussed above, the mechanical properties of the shell can be modified by adjusting the properties of the amphiphilic molecules, the metal cation, and the solvent conditions like pH, temperature or ionic strength. Adjustment of these conditions impacts the degree to which the amphiphilic molecules cross link in the shell and this degree of cross-linking impacts the mechanical properties.

By increasing the degree of cross-linking, it is estimated that the mesh size of the cross-linked molecules decreases which can increase the stability of the shell and/or decrease its permeability.

Increasing the number of metal coordinating groups on the amphiphilic molecules can increase the degree of cross-linking, associated with a corresponding increase in the number of available ions. Furthermore, selection of particular metal coordinating groups will also increase the degree and strength of cross-linking.

Modifying the metal cation will also impact the cross-linking and the mechanical properties of the shell by affecting the dissipation times of the complex-ion pair. A preferred metal cation is Fe³⁺ iron (III) but any suitable cation can be used.

Modifying the hydrophobic group(s) and/or hydrophilic group(s) can enable tuning of the shell's properties. For example, modification can influence the shell thickness, the mechanical stability (rigidity) and/or permeability.

Varying the ratio of metal-coordinating group to cation will impact the cross-linking and therefore the mechanical properties and permeability of the shell. This ratio can be impacted by the stoichiometric ratio of metal-coordinating group and cation present in the system. The ratio is also affected by pH. For example, for an amphiphilic molecule with catechol as metal-coordinating group and iron (III) as metal cation, at lower pH the molecules form a 1:1 mono structure as shown in figure 1a. As the pH increases, a 2:1 cross-linked bis-structure forms as shown in figure 1b. As the pH is increased yet further, a 3:1 tris cross-linked structure forms as shown in figure 1c. For example, it has been shown that for the Fe 3+ and dopamine system, at pH below 5.6 a mono structure forms, at pH= 5.6-9.1 a bis structure, and at pH>9.1 a tris structure. Thus, the ideal stoichiometric ratio of metal-coordinating group to cation will vary and can be tuned to modify the properties of the shell. The preferred ratio will depend on the desired properties of the shell. Maximising the ratio will form a strong shell, for example utilising a 3:1 catechol to metal ion where the ion allows for tris-coordination. However, if a shell with more flexibility is desired the ratio may be lower, for example 2:1. However, if a metal oxide or nanoparticle is used then the ratio may be significantly higher since many more metal-coordinating groups can coordinate with a single particle.

As previously discussed, by modifying the pH of the system it is possible to affect cross-linking and to change the degree of cross-linking. As such, it is possible to tune the properties of the shell by modifying the pH. It also makes shells according to the present invention sensitive to pH. Such shells find use as, for example, drug delivery vehicles which are sensitive to pH changes enabling precise drug delivery depending on conditions. Preferred pH ranges include 1-14, 5-14, 9-14, 10-14, 9-12, 10-12, around 9, around 12, 12-14.

It is possible to make the shell dissociate by varying parameters like pH or temperature. The addition of chelates such as but not limited to EDTA, EDDA, DTPA, HEDTA may also cause dissociation of the shell as the chelate traps the metal ion thereby affecting shell cross-linking.

It is also possible to cause the shell to irreversibly bind. This can be achieved by, for example, raising pH to a high pH which causes irreversible bonds to form. Such an approach may have advantages where it is no longer desired to have a self-healing reversible shell.

The self-healing shell of the present invention may be present in a system comprising the first fluid phase, the second fluid phase (and any further phases) and a self-healing shell at the interface between the phases.

The first fluid phase may be a liquid phase. This provides for a liquid/liquid phase system. The system may be an emulsion. In this embodiment, the first liquid phase may be an aqueous phase and the second liquid phase may be a non-aqueous phase. In a further embodiment, the first liquid phase may be a non-aqueous phase and the second liquid phase may be an aqueous phase. In a yet further embodiment, the first liquid phase and the second liquid phase may both be aqueous phases. In a yet further embodiment, the first liquid phase and the second liquid phase may both be non-aqueous phases. For example, in in a fluorinated / non fluorinated organic solvent.

It is possible to produce water-in-oil emulsions and oil-in-water emulsions according to the present invention. As such, it is possible to encapsulate an oil phase from an aqueous solvent or to encapsulate a water phase from an oil solvent. Also encompassed within the present invention are water-in-oil-in-water systems and oil-in-water-in-oil systems. Further emulsions are also possible including triple emulsions, multiple emulsions, double emulsions with multiple cores and the like.

In an alternate embodiment, the first fluid phase may be a gas phase. This provides for a liquid/gas phase system. The system may be a foam.

Suitable aqueous solvents include water. Further suitable aqueous solvents include low molecular weight poly(ethylene glycol) which is liquid under atmospheric conditions at suitable temperatures.

Suitable non-aqueous solvents may include but are not limited to aliphatic solvents such as hexane, decane, dodecane, hexadecane, alcohols, such as ethanol, methanol, hexanol, decanol, dodecanol, hexadecanol, alkenes, toluene, perfluorinated oils, fluorinated oils, perfluorocarbons, perfluoropolyethers, chloroform, ethers, dimethylformaide, dimethylsulfoxide, dichlormethane, pentane, cyclopentane, acetonitrile, isopropanol, methoxy-nonafluorobutaneethyl acetate, mineral oil, silicon-based oils, food grade oils including fish oil, sunflower oil, olive oil. Particular fluorinated products include 3M Fluorinert such as FC-40, 3M Novec engineering fluid such as Novec 7500 or Novec 7100.

The self-healing shells as described herein can be made by forming a system comprising an interface between a first fluid phase and a second fluid phase, amphiphilic molecules as defined herein, and metal cations as defined herein; wherein the first fluid phase is a liquid phase or a gas phase and the second fluid phase is a liquid phase; such that the metal-coordinating group(s) and metal cation(s) reversibly cross-link at the interface to form a self-healing shell.

The system may contain a third phase or further phases.

The first fluid phase may be a liquid. The first fluid phase may be an aqueous phase and the second fluid phase may be a non-aqueous phase. The first fluid phase may be a non-aqueous phase and the second fluid phase may be an aqueous phase. The first liquid phase and the second liquid phase may both be aqueous phases. The first liquid phase and the second liquid phase may both be non-aqueous phases. The first fluid phase may be a gas. The first fluid phase may be a gas and the second fluid phase may be an aqueous phase. The first fluid phase may be a gas and the second fluid phase may be a non-aqueous phase.

The interface may be formed by any suitable means. The interface will from when two phases are brought into contact with sufficient interfacial tension. Suitable means include adding one phase into the other phase, including dropwise addition; emulsifying the first phase and the second phase, including high pressure emulsification; microfluidics; or the use of a membrane.

For example, the first phase may be added dropwise into the second phase. By introducing a drop into the second phase, an interface is created between the two phases. At this interface, the amphiphilic molecules will adsorb, lowering the interfacial tension. At an appropriate pH, the metal-coordinating groups will reversibly cross-link with the metal cation and form a shell around the drop containing the first phase and shielding it from the second phase. The shell is self-healing because, in the event of shell rupture, the broken ionic bonds can reform and/or further amphiphilic molecules and cations present in the phases can coordinate to reform an intact shell.

The metal cations and amphiphilic molecules may be in the same phase or a different phase. Preferably, the metal cations and amphiphilic molecules are in a different phase. This helps to ensure that they only react at the interface.

In an embodiment, the metal cations may be in an aqueous phase and the amphiphilic molecules may be in a non-aqueous phase. In an embodiment, the metal cations may be in an aqueous phase and the amphiphilic molecules are in an aqueous phase. In an embodiment, the metal cations may be metal oxide or nanoparticles and may be in a non-aqueous phase and the amphiphilic molecules may be in an aqueous phase. In an embodiment, the metal cations may be metal oxide or nanoparticles and may be in an aqueous phase and the amphiphilic molecules may be in a non-aqueous phase. In a further embodiment, the metal cations may be metal oxide or nanoparticles and may be in a non-aqueous phase and the amphiphilic molecules may be in a second non-aqueous phase that is immiscible in the first non-aqueous phase.

For example, the amphiphilic molecules and metal cation may be contained in the first phase. Upon addition of the first phase into the second phase, an interface is created which causes the amphiphilic molecules to self-assemble at the interface. The presence of the metal cations enables reversible cross-linking of the amphiphilic molecules. Thus, by addition of the first phase into the second phase, the first phase contains the components necessary to form the shell.

Alternatively, amphiphilic molecules and metal cation may be contained in the second phase, into which the first phase is added. In the same way as previously, an interface is created which causes self-assembly of the amphiphilic molecules and cross-linking but in this embodiment, the components necessary to form the shell are contained in the phase into which the first phase is added.

As a further preferred embodiment, the amphiphilic molecules may be contained in one phase and the metal cations contained in another phase. This way, the components do not interact until the phases are brought together and thereafter the components will react at the interface. In the same way as previously, an interface is created which causes self-assembly of the amphiphilic molecules and cross-linking but in this embodiment, the components necessary to form the shell come into contact at the interface.

As discussed, the ability to cross-link to form a shell is impacted by pH. An system can be prepared at one pH and the pH be subsequently adjusted to effect cross linking.

Once the shell has been formed, it is possible to remove fluid within the system to retain the intact shell. If the shell is encapsulating an inner liquid then this will retain intact thereby allowing the shell to be removed from the system.

It is also possible after formation to increase the pH such that irreversible bonds form within the shell thereby producing a covalently cross-linked shell. Such an approach does not require the presence of metal cations.

The shells of the present invention can be used to encapsulate a material and thereby to form a capsule.

In an embodiment, after formation of the self-healing shells of the present invention, for example capsules, the external phase can be removed or replaced leaving intact capsules containing the inner phase.

In an embodiment, it is possible to form layers of self-healing capsules followed by removal of the external phase to produce a hydrogel with unique biomechanical properties. By ionically linking adjacent capsules, thereby producing larger materials with well-defined compositions and locally varying compositions, advantageous materials can be prepared that mimic natural materials. The mechanical properties of the hydrogel can be tuned depending on the selection of materials used to make the shell, as well as the configuration of capsules both in terms of the orientation of the capsules in a layer, and the ability to stack layers on top of each other to form hydrogels with unique properties.

There are multiple uses for the self-healing shells according to the present invention. The shells can encapsulate material with a robust and self-healing shell. This allows materials to be protected. It also allows materials to be stored and/or selectively transported/moved. For example, it is desirable in many applications to be able to encapsulate a material into a first closed system and then manipulate the closed system to selectively allow the material to come into contact with a second closed system. This could be used, for example, to selectively allow a shell containing a fluid (liquid or gas) and defining a first closed system to come into contact with a second shell containing a second fluid and defining a second closed system. The self-healing nature of the shell enables the two shells to fuse and bring the first and second fluids into contact with each other forming a new shell that surrounds the newly formed mixed fluid. Such manipulations can be used for research on investigatory purposes.

As such, in an embodiment, there is provided a method of transporting material from a first closed system to a second closed system within a surrounding system using self-healing capsules described herein; comprising forming a first self-healing capsule comprising a shell to encapsulate a first fluid and form a first closed system; forming a second self-healing capsule comprising shell to encapsulate a second fluid and form a second closed system; bringing the first and second self-healing capsules into contact such that the first and second shells fuse bringing the first and second closed systems into contact with each other while still being protected from the surrounding system by the self-healing shell.

Such a system allows for the controlled transportation of material. This material could be drugs, food ingredients, nutraceuticals, cosmetics, pesticides, nutrients, fragrances, catalysts, agrichemicals, reagents to form proteins, coatings, paints, waste products and the like. While the material within each closed system can be mixed and fused, it does not come into contact with the surrounding system since it is always encapsulated by the shell.

A specific embodiment is the use of self-healing shell as described herein in droplet based screening assays. By forming self-healing shells around target material, cross-talk between droplets caused by material leakage is reduced, improving the accuracy of the assay. This allows for more sensitive measurements since the target analyte and any reagents can be contained within the shell and will not leak into the surrounding solvent or another drop. Particular screening uses include high-throughput screening, high throughput drug screening, high throughput antibody screening, or single cell sequencing.

Droplet based screening assays rely on each water in oil or water in oil in water drop to be a closed container to conduct a biological or chemical reaction. Common used surfactants which stabilize the emulsion drops can also facility the transport of material in form of aggregates or tiny emulsion drops through the oil phase possible from one drop to another which results in less accurate assays. This transport of material through the liquid/liquid interface can be reduced by cross-linking the interface as described herein.

The self-healing shells according to the present invention may be used as a vehicle for drug delivery or for the encapsulation of drugs, food ingredients, nutraceuticals, cosmetics, pesticides, nutrients, fragrances, catalysts, agrichemicals, reagents to form proteins, coatings, paints, waste products and the like. Due to their pH sensitive nature, the shells according to the present invention are particularly suited to situations where pH controlled release is desired. The shell can also show temperature sensitivity enabling temperature controlled release.

The shells of the present invention may be used to produce high stability emulsions or foams. An advantage of these emulsions or foams is their ability to withstand changes to osmotic pressure or other mechanical forces.

In a yet further embodiment, there is provided an amphiphilic molecule as defined herein comprising one or more hydrophilic group(s) and one or more hydrophobic group(s); and wherein the amphiphilic molecule comprises one or more metal-coordinating group(s); wherein the amphiphilic molecule is a block copolymer; and wherein the metal-coordinating group is a metal-coordinating group selected from the group consisting of: benzenediol or derivatives thereof, preferably catechol or derivatives thereof; and benzenetriol or derivatives thereof, preferably gallol or derivatives thereof; and wherein the metal-coordinating group may optionally be further substituted.

Such amphiphilic molecule have not previously been identified and allow for the formation of self-healing shells as described herein when coordinated with a metal cation.

The hydrophilic group(s), hydrophobic group(s), and metal-coordinating group(s) may be as defined herein.

The invention will now be described by way of the following non-limiting examples.

### Examples

### Synthesis of FSHDopa

The fluorinated surfactant (FSHDopa) was synthesized following previously published work: Holtze, C. et al. Biocompatible surfactants for water-in-fluorocarbon emulsions. Lab Chip 8, 1632-1639 (2008). One equivalent of the perfluorinated polyether Krytox FSH 157 (-6500 g/mol, Chemours, USA) was dissolved in Novec HFE-7100 (3M, USA) under Argon. 10 mol equivalent of thionylchloride (Sigma-Aldrich, USA) was added to the clear solution and refluxed at 65 °C for 2 hours to activate carboxylic acid end group of the Krytox by transforming it into an acid chloride. The excess solvent and excess thionylchloride is subsequently removed by keeping the solution at 90 °C and a reduced pressure for 1h. After the activated Krytox has cooled to room temperature it was redispersed in HFE-7100 at 0.1 g/ml. To this solution of anhydrous dimethyl formamide (abcr, Germany) containing 4 mol equivalent (0.05 g/ml) of Dopamine HCI (Sigma-Aldrich, USA) was cooled to 0°C before 6 mol equivalent of trimethylamine (Sigma-Aldrich, USA) was added. This solution was stirred at room temperature for 30 min before it was added to the activated Krytox under inert atmosphere. The reaction was stirred at 65 °C overnight. The product was filtered through a filter paper, put in a separating funnel and washed three times with water and HFE-7100 .

### Synthesis of FSHPEG900HA

This synthesis was performed in 3 steps. The Krytox FSH was activated using thionylchloride as explained above following protocols that have previously been published in Etienne, G., Kessler, M. & Amstad, E. Influence of Fluorinated Surfactant Composition on the Stability of Emulsion Drops. Macromol. Chem. Phys. 218, 1-10 (2017). The Jeffamine diamine (hydrophilic group) was coupled to the Krytox and purified as described. Subsequently, 1 mol equivalent Hydrocaffeic acid (abcr, Germany) was dissolved in DMF at 0.005 g/ml and cooled to 0°C before 1.1 mol equivalent of N-Hydroxysuccinimide (NHS, TCI, Japan) and 1.1 mol equivalent of N,N-Dicyclohexylcarbodiimid (Sigma-Aldrich, USA) were added. This solution was stirred for 2h at 0°C before it was heated to room temperature and stirred at this temperature for 1h. The FSHPEG900-amine was added to this solution at 0.5 mol equivalent (0.04 g/ml) and the mixture was stirred overnight before the solution is filtered and the solvents are removed in the rotary evaporator. We purified the product with a 10:1 methanol:HFE7100 mixture before the product is dried in vacuum. Additionally the reaction can also be done by first linking the hydrocaffeic acid to the PEG by DCC/NHS coupling and then linking it to the fluorinated Krytox FSH 157.

To increase the size of the hydrophilic part, a PEG-spacer was added that separates the fluorinated block from the catechol.

### Synthesis of SA-Dopa (N-Stearoyldopamine)

1 mol equivalent of stearoyl chloride (TCI, Japan) was dissolved in anhydrous dimethyl formamide (abcr, Germany) at 0.05 g/ml under dry conditions. 1.5 mol equivalent of Dopamine HCI (Sigma-Aldrich, USA) was dissolved in anhydrous DMF at 0.2 g/ml under argon. While cooling the solution to 0°C 3 mol equivalent of trimethylamine (Sigma-Aldrich, USA) was added to the DMF-based solution containing dopamine. This solution was subsequently added to the solution containing stearoyl chloride and stirred overnight at 65 °C under inert atmosphere. The solution was filtered while it was before the DMF was removed using a Rotary evaporator. The product was purified using water and ethyl acetate (Sigma-Aldrich, USA). The washing was repeated 3 times and before the product was washed once with 1M HCl to remove the excess trimethylamine.

### Synthesis of Oleic acid-DOPA

1 mol equivalent of oleic acid is dissolved in dry chloroform at 0.1 g/ml

1 mol equivalent of oleic acid (Sigma-Aldrich, USA) was dissolved in anhydrous chloroform (Sigma-Aldrich, USA) at 0.1 g/ml under dry conditions. 10 mol equivalent of thionylchloride (Sigma-Aldrich, USA) was added and stirred for 2h at 60°C. 3 mol equivalent of Dopamine HCl (Sigma-Aldrich, USA) was dissolved in anhydrous DMF at 0.2 g/ml under argon. This solution was subsequently added to the solution containing stearoyl chloride and stirred overnight at 65°C under inert atmosphere. When mixed together 3 mol equivalent of trimethylamine (Sigma-Aldrich, USA) was added to the mixture. The solution was filtered while it was before the DMF was removed using a Rotary evaporator. The product was purified using water and chloroform (Sigma-Aldrich, USA).

The unsaturated bond in the hydrobarbon chain changes the stiffness of the molecule and therefore its packing density and propensity to form aggregates

### Synthesis of DiDopa-PPG:

This synthesis of DiDopa-PPG was performed using protocols established for the synthesis of FSHDopa. Instead of using a fluorinated solvent (HFE-7100) we employed chloroform (for the activation) and dimethyl formamide (for the coupling reaction). The extraction was done with diethyl ether and water.

### Example 1 Behaviour of surfactants at water-oil interface

To observe the behaviour of the surfactants at the water-oil interface, oil containing the surfactants was injected into a water bath, as shown in Figure 2.

When the surfactant is not crosslinked because the outer solution is a low pH and the liquid is retracted, the drop shrinks as seen in Figure 2a. In contrast, when the surfactants are cross-linked by simultaneously shifting the pH to slightly basic conditions, a shell is formed at the interface as seen in Figure 2b. When liquid is retracted from these drops, the shell buckles as seen in the time lapse micrographs in Figure 2b. When oil is re-introduced into the drops, the drop recovers, as shown in the time lapse micrographs in Figure 2c.

Figure 2a shows a microscope image of 1 wt.% of FSHDopa dissolved in fluorinated oil HFE 7100 containing Fe 3+ in water during removal or injection of oil. The water bath contains 10 mM HCI solution. The FSHDopa is not cross-linked and it can be seen that when the liquid of the drop is removed the drop correspondingly shrinks in size.

Figure 2b shows the 1 wt.% of FSHDopa dissolved in fluorinated oil HFE 7100 drop in a water bath (at pH=7.7) containing FeCl₃ for 3:1 catechol to iron ratio. Under these conditions, the catechol undergoes a strong linkage with the ions and the surfactant forming a viscoelastic interface. When the oil is retracted from the drop the drop starts to buckle strongly.

Figure 2c shows oil in reinjected into the buckled drop, and it can be seen that the drop fully recovers to its initial spherical shape.

### Example 2 Liquid exchange between drops

An oil phase (HFE 7100) containing 1 wt.% FSHDopa and 1:2 catechol:Fe by adding 1M FeCI3 in EtOH to the surfactant solution is prepared. The outer water phase was at pH=7.7. First a drop of oil solution is deposited on the bottom of the cuvette. Next, a second drop is formed at the needle by injecting oil phase and retracting it.

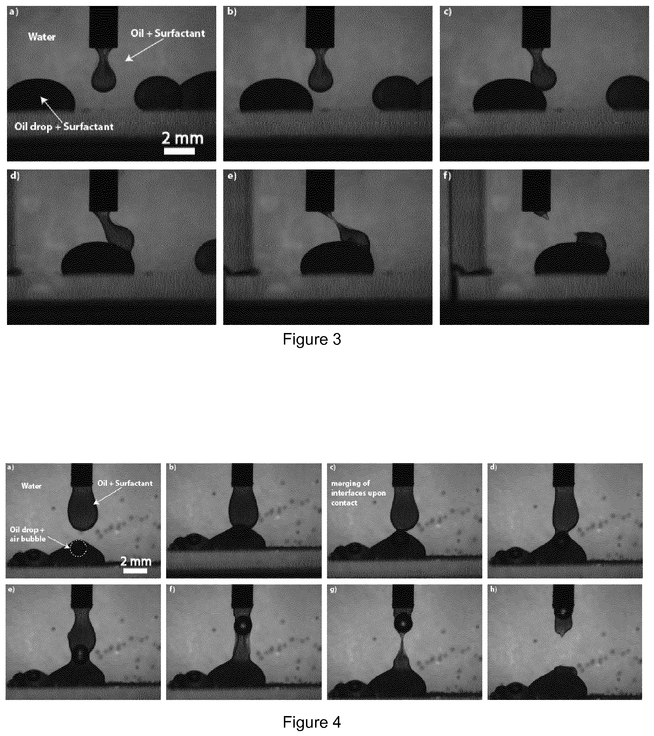

When two oil drops comprising the shells made from the ionically crosslinked surfactants of the present invention are brought in contact, the drops fuse and allow exchange of liquids contained in them, as exemplified in Figure 3.

Figures 3a-c show microscope images of oil and surfactant drops (HFE 7100 + 1 wt.% FSHDopa and 3:16 FeCI3 added to oil phase) in water, in which two drops are brought into contact. The drop attached to the needle is slowly moved towards a drop sitting at the bottom of the glass cuvette.

Upon contact of the two interfaces (Figure 3d), the two interfaces merge into one interface (Figures 3e-f). As the sample is further moved, the connection ultimately rips of. It can be seen in the last image that the new added drop partially keeps its shape after ripping off from the needle.

Example 2 demonstrates the use of the shells of the present invention in the controlled transportation of reagents, nutrients, and waste products between different drops.

### Example 3 Transportation of material between closed containers

An oil phase (HFE 7100) containing 1 wt.% FSHDopa and 1:2 catechol:Fe by adding 1M FeCI3 in EtOH to the surfactant solution was prepared. The outer water phase was at pH=7.7. First a drop of oil solution is deposited on the bottom of the cuvette, the drop contains a air bubble as a model system to transport. Next, a second drop is formed at the needle by injecting oil phase and retracting it.

Viscoelastic capsules of high stability and with self-healing properties were fabricated and allow for transportation of material from one closed container to another as shown in Figure 4.

An air bubble (used as a model) is trapped in an oil drop on the surface of a glass vial. The air bubble can be seen in the drop on the surface of the glass cuvette in Figure 3a. When a second drop is placed in contact with the first drop, the two interfaces merge and the air bubble is transported into the other drop without getting in contact with the surrounding water phase. This can be seen in from the second drop (hanging from the needle) approaching the first drop (Figures 4b-c); the two interfaces merge upon contact and the bubble moving from one drop to another (Figures 4d-f); and then being detached (Figure 4h).

### Example 4 Use of capsules in screening assays

Double emulsions are formed using a microfluidic device. To form these double emulsions, an aqueous phase containing 10 wt.% PVA (for responsive test at pH = 8.5) was used as an outer phase and a perfluorinated oil (HFE7100) containing 1 mM FSHPEG900HA (responsive) or 1 mM DiFSHPEG900 (non responsive surfactant) was employed as a middle phase and an aqueous phase containing 20 wt. PEG + 0.1 wt% Fluorescein was employed as an inner phase. After producing the double emulsions they are washed with a water and sucrose solution at equal osmotic pressure as the inner phase and for the responsive surfactant adapted to pH=8.5. The leakage of the dye over times is observed with the responsive compared to the non-responsive surfactant.

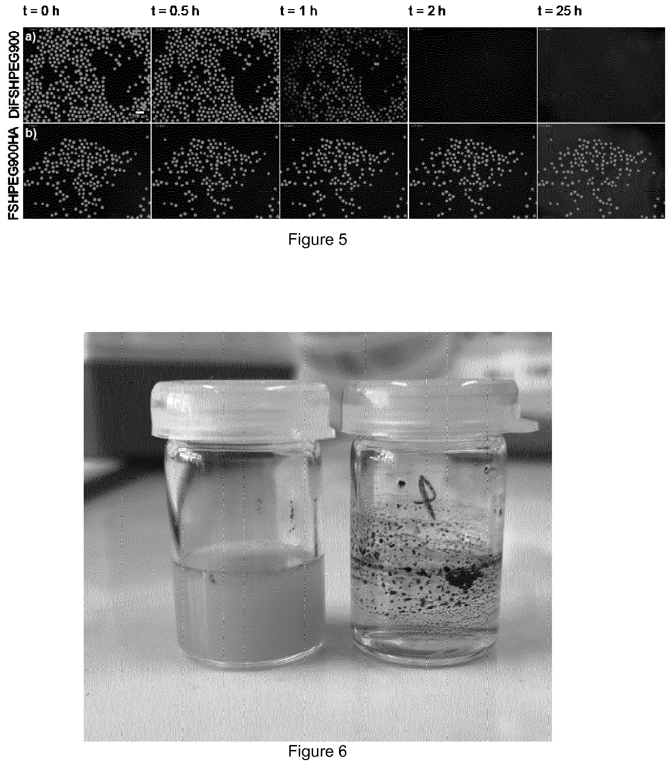

A water in oil in water double emulsions containing fluorescein as a dye in the inner phase was produced. The leakage of fluorescein dye from double emulsions stabilized with a non-functionalized fluorinated surfactant (DiFSHPEG9000) was analysed by observing them under fluorescent microscope over time. It was observed that after 2 hours most of the dye had leaked out of the double emulsion as seen in Figure 5a.

In contrast, when the same experiment was performed using capsules of the present invention using surfactant (FSHPEG900HA) cross-linked with iron, leakage was drastically reduced as shown in Figure 5b. It can be seen that after 25h, almost no leakage was seen.

By preparing capsules of the present invention, cross-talk between drops was reduced thereby improving the accuracy of screening assays.

### Example 5: Catechol-functionalized perfluorinated block-copolymers

To verify that catechols are coupled to the perfluorinated block-copolymers, the block-copolymer is dissolved in a fluorinated solvent (HFE7100). FeCl₃ is dissolved in ethanol and added to the surfactant containing solution. Because the catechol is bound to the perfluorinated block-copolymers, it is soluble in the fluorinated solvent such that it forms complexes with the Fe³⁺ ions and the solution becomes green, as shown on the left side of Figure 6. By contrast, if free catechols are added to the fluorinated solvent and Fe³⁺ ions are added, green precipitates form, as shown on the right side of Figure 1. In this case, catechols are insoluble in the fluorinated solvent such that they precipitate to the walls of the vial. Upon addition of Fe³⁺, the precipitates form complexes that become green. However, they cannot be dispersed in the fluorinated solvent because they are insoluble.

### Example 6: influence of pH on interfacial tension

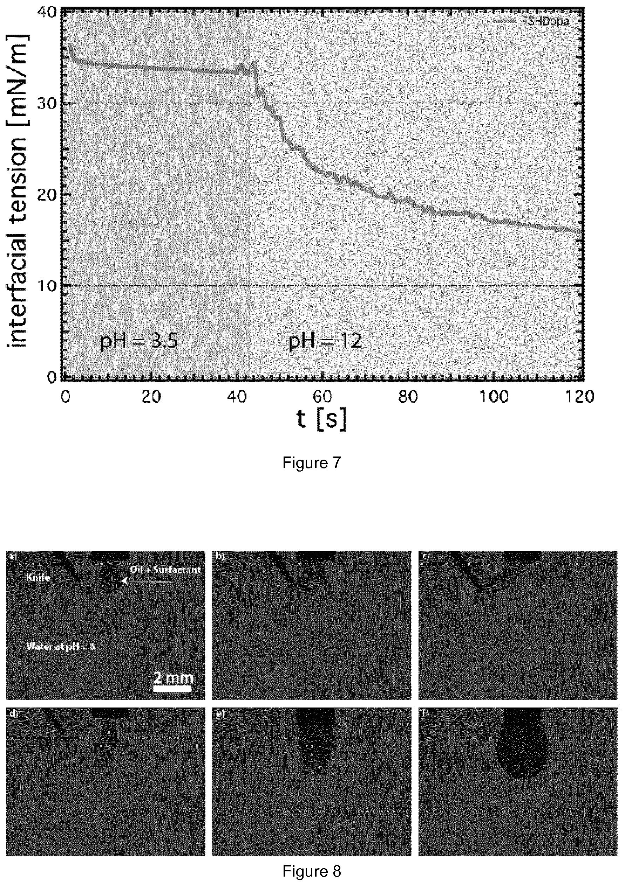

To test the influence of the pH on the interfacial tension between a fluorinated solvent, HFE7500 containing 1 wt% FSHDopa and water, we perform pendant drop measurements. If the pH is low such that most of the catechols are protonated, the interfacial tension is high, approximately 35 mN/m. By contrast if we increase the pH above the pKa values of DOPA, the interfacial tension strongly decreases, as shown in Figure 7.

### Example 7: testing self-healing behaviour

To test if the shells composed of FSHDOPA/Fe3+ complexes display a self-healing behaviour, we form a drop composed of HFE7100 that contains 1wt% FSHDopa. The drop is immersed in an aqueous solution containing Fe3+. We observe the formation of a thin shell at the liquid-liquid interface, as shown in Figure 8a. The shell has an affinity to iron-containing surfaces such that it adheres to a steel knife if the knife is brought in its proximity, as shown in Figure 8b-c. The shell adheres to the knife and can be mechanically deformed up to the point where it breaks. When the knife is retracted, a part of the shell remains attached to the knife and is separated from the rest of the shell, as shown in Figure 8d. With time, the remaining shell self-heals, as shown in Figures 8e-f. In Figure 8e the shell self-heals and in 8f upon re-injection of additional oil, the shell becomes again spherical.

### Example 8: influence of hydrophobic chain

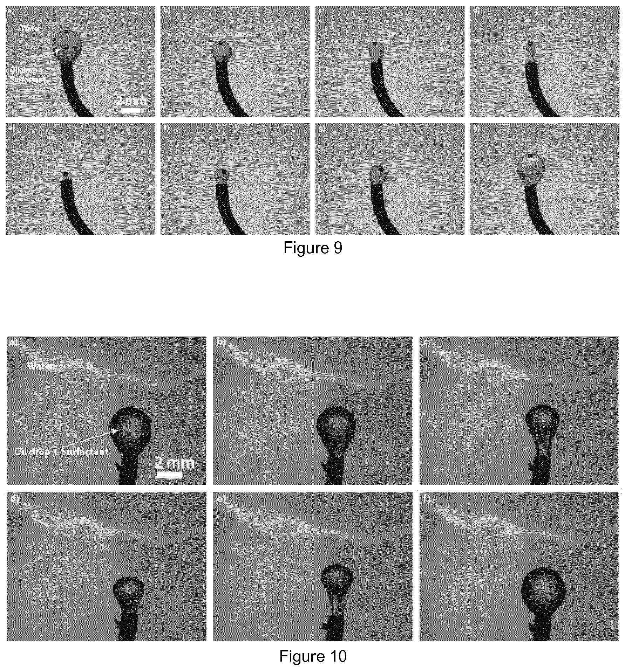

To test the influence of the hydrophobic chain of catechol-functionalized surfactants on their ability to form self-healing shells, we synthesized a surfactant composed of stearic acid that is linked to a catechol. This surfactant was dissolved in ethyl acetate containing Fe³⁺ ions and the solution was used to form a drop that is surrounded by an aqueous phase (pH = 7.7). Also in this case, we observe that a shell that buckles forms at the liquid-liquid interface, as indicated by the time lapse micrographs in Figure 9.

Similarly, we observed the formation of a shell if a surfactant composed of two catechols that are interspaced by polypropylene glycol) (diDOPA-PPG) is dispersed in toluene containing Fe³⁺ and this phase is used to form a drop in an aqueous phase as shown in the time lapse micrographs in Figure 10

### Example 9: influence of hydrophobic chain

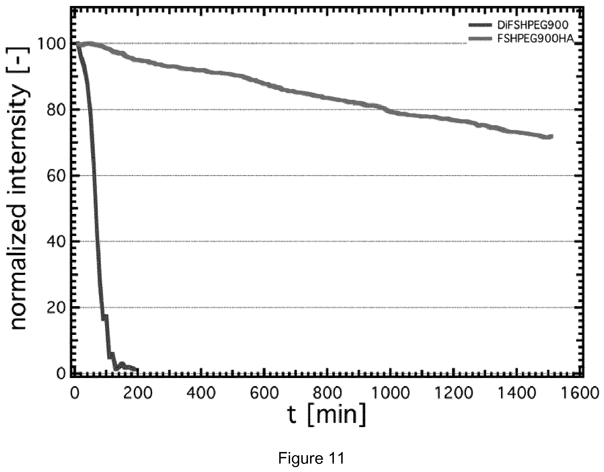

To test the influence of the surfactant on the permeability of water-oil-water double emulsions, we formed double emulsions composed of an aqueous core, a perfluorinated shell, and a surrounding aqueous phase. The diameter of the double emulsion was approximately 90 µm, the shell thickness was approximately 10 µm. To quantify the permeability, we loaded fluorescein into the core of the double emulsions. We observed that double emulsions stabilized with non-functionalized surfactants, such as DiFSHPEG900 were highly permeable and the majority of fluorescein was released within 100 min. By contrast, if double emulsions were stabilized with the catechol-modified surfactant, FSHPEG900HA and Fe3+ ions were added to link these surfactants, thereby forming a shell, only a very small fraction of fluorescein was released within 100 min. Indeed, these double emulsions retained the vast majority of the encapsulants for more than 1500 min, as shown in Figure 11.

In conclusion, self-healing shells according to the present have been identified which offer new opportunities. The presence of metal coordinating groups and metal ions allows for the formation of self-healing shells which have tunable mechanical properties and can advantageously be used in a number of applications including as encapsulants, novel materials, and in the pharmaceutical, screening, agrichemical, fragrance, coatings, food and other sectors.

The invention will now be described in the following clauses:
1. A self-healing shell at an interface between a first fluid phase and a second fluid phase; wherein the first fluid phase is a liquid phase or a gas phase and the second fluid phase is a liquid phase; said shell comprising amphiphilic molecules and metal cations; wherein the amphiphilic molecules comprise one or more hydrophilic group(s) and one or more hydrophobic group(s), and wherein the amphiphilic molecules comprise one or more metal-coordinating group(s); wherein the metal cations comprise metal ions, metal oxides, metal hydroxides, metal carbides, metal nitrides and/or metal nanoparticles; and wherein amphiphilic molecules in the shell are reversibly cross-linked via the one or more metal-coordinating group(s) and metal cations.
2. The self-healing shell according to clause 1, wherein the shell is a viscoelastic shell.
3. The self-healing shell according to any one of clauses 1 to 2, wherein the shell is a capsule or a membrane.
4. The self-healing shell according to any one of clauses 1 to 3, wherein the first fluid phase is a liquid phase; and wherein there may optionally be further phases.
5. The self-healing shell according to clause 4, wherein the first liquid phase is an aqueous phase and the second liquid phase is a non-aqueous phase; wherein the first liquid phase is a non-aqueous phase and the second liquid phase is an aqueous phase; wherein the first liquid phase and the second liquid phase are both aqueous phases or wherein the first liquid phase and the second liquid phase are both non-aqueous phases.
6. The self-healing shell according to clause 4, wherein the first liquid phase and the second liquid phase form an emulsion; wherein the emulsion may be a water-in-oil emulsion; an oil-in-water emulsion; a water-in-oil-in-water emulsion; an oil-in-water-in-oil emulsion; a triple emulsion; a multiple emulsion; or a double emulsion with multiple cores.
7. The self-healing shell according to any one of clauses 1 to 3, wherein the first fluid phase is a gas phase; and wherein the second liquid phase may optionally be an aqueous phase or a non-aqueous phase.
8. The self-healing shell according to clause 7, wherein the first gas phase and the second liquid phase form a foam.
9. The self-healing shell according to any one of clauses 1 to 8, wherein the aqueous phase or phases has a pH in the range of 1-14, preferably 5-14, 9-14, 10-14, 5.5-12, 7.5-12, 9-12, 10-12, around 9, around 12, 12-14.
10. The self-healing shell according to any one of clauses 1 to 9, wherein the metal-coordinating group is selected from the group consisting of: benzenediol or derivatives thereof, preferably catechol or derivative thereof; benzenetriol or derivatives thereof, preferably gallol or derivatives thereof; histidines and derivatives thereof; groups comprising a carboxyl group; ethylenediaminetetraacetic acid and derivatives thereof; and wherein the metal-coordinating group may optionally be further substituted.
11. The self-healing shell according to clause 10, wherein hydroxyl groups are present in the ortho-meta position or meta-para position relative to the amphiphilic molecule; preferably the meta-para position.
12. The self-healing shell according to any preceding clause, wherein there is one metal coordinating group per amphiphilic molecule; two metal coordinating groups per amphiphilic molecule; three metal coordinating groups per amphiphilic molecule; more than three metal coordinating groups per amphiphilic molecule; a metal coordinating group is incorporated into the repeating unit of a polymer.
13. The self-healing shell according to any preceding clause, wherein the metal-coordinating group(s) is/are attached to hydrophilic group(s) and/or wherein the metal-coordinating group(s) is/are attached to hydrophobic group(s).
14. The self-healing shell according to any preceding clause, wherein the metal-coordinating group(s) form a/the hydrophilic group(s).
15. The self-healing shell according to any preceding clause, wherein the one or more hydrophilic group(s) include hydrophilic polymer(s); wherein the hydrophilic polymer(s) may optionally be selected from polyethyleneglycol (PEG), polyacrylicacid (PAA), polyethyleneimine (PEI), polyvinylalcohol (PVA), Poly(N-isopropylacrylamide) (PNIPAM), poly(2-methyl-2-oxazoline) (PMOXA), polyglycols, natural hydrophilic polysaccharides including dextran, alginate, and peptides.
16. The self-healing shell according to any preceding clause, wherein the one or more hydrophobic group(s) include: substituted or unsubstituted lipophilic hydrocarbon chains, fluorinated chains, perfluorinated polyether, hydrophobic polymer(s), polypeptides and/or liquid crystals.
17. The self-healing shell according to clause 16, wherein the one or more hydrophobic group(s) include lipophilic hydrocarbon chains; wherein the lipophilic hydrocarbon chains are preferably branched or unbranched having from 4 to 18 carbon atoms; wherein the lipophilic hydrocarbon chain may be saturated or unsaturated; and wherein the lipophilic hydrocarbon chain may optionally be substituted with one or more functional groups, particularly stearic acid, oleic acid, saturated hydrocarbon.
18. The self-healing shell according to clause 16, wherein the one or more hydrophobic group(s) include hydrophobic polymer(s); wherein the hydrophobic polymer(s) may be selected from acrylics, amides and imides, carbonates, dienes, esters, ethers, fluorocarbons, perfluorinated polyethers, olefins, styrenes, vinyl acetals, vinyl and vinylidene chlorides, vinyl esters, vinyl ethers and ketones, vinylpyridine and vinypyrrolidone polymers;
   wherein the hydrophobic polymer(s) are more preferably selected from polypropyleneglycol (PPG), polystyrene (PS), polylacticacid (PLA), fluorocarbons, methacrylates, polyethylene, polydimethylsiloxane (PDMS), chitosan and cellulose;
   wherein the hydrophobic polymer(s) are particularly preferably selected from fluorocarbons including perfluorinated polyethers, particularly perfluorinated polyethers with carboxylic acid-, methyl ester-, methylene alcohol- or allyl ether end groups. Krytox FSH 157, Krytox FSM 157, Krytox FSL 157, FC40.
19. The self-healing shell according to any preceding clause, wherein the amphiphilic molecules comprise block copolymers;
   wherein the block copolymers may be selected from: diblock copolymers, triblock copolymers, and/or random block copolymers;
   wherein the diblock copolymers may optionally be selected from: AB diblock copolymers, PEG diblock copolymers, polystyrene diblock copolymers;
   wherein the triblock copolymers may optionally be selected from: ABA triblock copolymers, ABC triblock copolymers, biodegradable triblock copolymers, PEG/PPG triblock copolymers, polystyrene triblock copolymers, multi-arm PEG block copolymers, PNIPAM-based block copoylmers, PIMOXA based block-copolymers, light responsive block copolymers, temperature responsive block copolymers Catechol-PPG-Catechol,Catechol-perfluorinated polyether-catechol, perfluorinated polyether-PEG-Catechol, polypeptides; and/or wherein the random block copolymers may optionally be selected from any combination of the blocks above, provided at least one block is hydrophobic and at least one block is hydrophilic.
20. The self-healing shell according to any preceding clause, wherein the amphiphilic molecule is a bipolar amphiphilic molecule.
21. The self-healing shell according to any preceding clause, wherein the metal cations may be selected from the group consisting of:
   metal ions selected from Be²⁺ beryllium ion, Mg²⁺ magnesium ion, Ca²⁺ calcium ion, Sr²⁺ strontium ion, Ba²⁺ barium ion, Ti²⁺ titanium (II), Ti⁴⁺ titanium (IV), Cr²⁺ chromium (II), Cr³⁺ chromium (III), Cr⁶⁺ chromium (VI), Mn²⁺ manganese (II), Mn³⁺ manganese (III), Mn⁴⁺ manganese (IV), Fe²⁺ iron (II), Fe³⁺ iron (III), Co²⁺ cobalt (II), Co³⁺ cobalt (III), Ni²⁺ nickel (II), Ni³⁺ nickel (III), Cu⁺ copper (I), Cu²⁺ copper (II), Ag⁺ silver ion, Au⁺ gold (I), Au⁺³ gold (III), Zn²⁺ zinc ion, Cd²⁺ cadmium ion, Hg₂²⁺ mercury (I), Hg²⁺ mercury (II), Al³⁺ aluminium ion, Ga³⁺ gallium ion, In⁺ indium (I), In³⁺ indium (III), Sn²⁺ tin (II), Sn⁴⁺ tin (IV), Pb²⁺ lead (II), Pb⁴⁺ lead (IV), Bi³⁺ bismuth (III), and/or Bi⁵⁺ bismuth (V); preferably iron, aluminium or titanium; and most preferably iron;
   metal oxides, metal carbides, metal nitrides, and/or
   metal nanoparticles including iron oxide, iron nitrides, iron carbides, nickel oxides, nickel carbides, titanium oxides, titanium metal particles, titanium nitrides and titanium carbides.
22. A method of manufacturing self-healing shells from any one of clauses 1 to 21;
   the method comprising forming a system comprising: an interface between a first fluid phase and a second fluid phase, amphiphilic molecules as described in any one of clauses 10 to 20 and metal cations from clause 21;
   wherein the first fluid phase is a liquid phase or a gas phase and the second fluid phase is a liquid phase;
   such that the metal-coordinating group(s) and metal cation(s) reversibly cross-link at the interface to form a self-healing shell.
23. The method according to clause 22, wherein the interface is formed by: adding one phase into the other phase, including dropwise addition; emulsifying the first phase and the second phase, including shearing, high pressure emulsification; microfluidics; or the use of a membrane.
24. The method according to clause 22 or 23, wherein the first fluid phase is a liquid; wherein the first fluid phase is an aqueous phase and the second fluid phase is a non-aqueous phase; wherein the first fluid phase is a non-aqueous phase and the second fluid phase is an aqueous phase; wherein the first liquid phase and the second liquid phase are both aqueous phases; wherein the first liquid phase and the second liquid phase are both non-aqueous phases; wherein the first fluid phase is a gas; wherein the first fluid phase is a gas and the second fluid phase is an aqueous phase; or wherein the first fluid phase is a gas and the second fluid phase is a non-aqueous phase.
25. The method according to any one of clauses 22 to 24, wherein the metal cations and amphiphilic molecules are in the same phase or a different phase; preferably wherein the metal cations and amphiphilic molecules are in a different phase.
26. The method according to clause 25, wherein metal cations are in an aqueous phase and the amphiphilic molecules are in a non-aqueous phase; or wherein metal cations are in an non-aqueous phase and the amphiphilic molecules are in a aqueous phase; or wherein metal cations are in an non-aqueous phase and the amphiphilic molecules are in a non-aqueous phase; or wherein
   the metal cations are in an aqueous phase and the amphiphilic molecules are in an aqueous phase; or wherein the metal cations are metal oxide or nanoparticles and are in a non-aqueous phase and the amphiphilic molecules are in an aqueous phase; or wherein the metal cations are metal oxide or nanoparticles and are in a non-aqueous phase and the amphiphilic molecules are in a non-aqueous phase.
27. The method according to any one of clauses 22 to 26, wherein the pH of the system is adjusted to effect cross-linking or dissociation of the cross-linking.
28. The method according to any one of clauses 22 to 27, wherein the pH of the system is 1-14, preferably 5-14, 9-14, 10-14, 5.5-12, 7.5-12, 9-12, 10-12, around 9, around 12, 12-14.
29. The method according to any one of clauses 22 to 28, wherein after formation of the shell in the form of a capsule, the fluid on the exterior of the capsule is removed or replaced.
30. A method of transporting material from a first closed system to a second closed system within a surrounding solvent using self-healing shells as from any one of clauses 1 to 21; comprising forming a first self-healing capsule comprising a shell to encapsulate a first fluid and form a first closed system; forming a second self-healing capsule comprising shell to encapsulate a second fluid and form a second closed system; bringing the first and second self-healing capsules into contact such that the first and second fluids fuse bringing the first and second closed systems into contact with each other while still being protected from the surrounding solvent by the self-healing shell.
31. The method of clause 30, enabling the controlled transportation of drugs, food ingredients, nutraceuticals, cosmetics, pesticides, nutrients, fragrances, catalysts, agrichemicals, reagents to form proteins, coatings, paints, or waste products between different closed systems.
32. The use of a self-healing shell according to any one of clauses 1 to 21 in a droplet based screening assay.
33. The use according to clause 32, wherein the screening assay is high-throughput screening, where the screening can be but is not limited to high throughput drug screening, high throughput antibody screening, or single cell sequencing.
34. The use of self-healing shells according to any one of clauses 1 to 21 as a vehicle for drug delivery and/or for encapsulation of drugs, food ingredients, nutraceuticals, cosmetics, pesticides, nutrients, fragrances, catalysts, agrichemicals, reagents to form proteins, coatings, paints, or waste products.
35. The use according to clause 34, wherein release is controlled by pH and/or wherein release is controlled by temperature.
36. The shell according to any one of clauses 1 to 21, method according to any one of clauses 22 to 31, or use according to any one of clauses 32 to 35, wherein chelates are used to dissociate the shell; preferably EDTA, EDDA, DTPA, or HEDTA.
37. Three-dimensional hydrogel material comprising self-healing shells from any one of clauses 1 to 21.
38. An amphiphilic molecule as defined in any one of clauses 10 to 20, comprising one or more hydrophilic group(s) and one or more hydrophobic group(s); and wherein the amphiphilic molecule comprises one or more metal-coordinating group(s); wherein the amphiphilic molecule is a block copolymer; and wherein the metal-coordinating group is a metal-coordinating group selected from the group consisting of: benzenediol or derivatives thereof, preferably catechol or derivatives thereof; and benzenetriol or derivatives thereof, preferably gallol or derivatives thereof; histidines or derivatives thereof; and ethylenediaminetetraacetic acid and derivatives thereof; and wherein the metal-coordinating group may optionally be further substituted.

## Claims

1. A self-healing shell at an interface between a first fluid phase and a second fluid phase; wherein the first fluid phase is a liquid phase or a gas phase and the second fluid phase is a liquid phase; said shell comprising amphiphilic molecules and metal cations; wherein the amphiphilic molecules comprise one or more hydrophilic group(s) and one or more hydrophobic group(s), and wherein the amphiphilic molecules comprise one or more metal-coordinating group(s); wherein the metal cations comprise metal ions, metal oxides, metal hydroxides, metal carbides, metal nitrides and/or metal nanoparticles; and wherein amphiphilic molecules in the shell are reversibly cross-linked via the one or more metal-coordinating group(s) and metal cations.

2. The self-healing shell according to claim 1, wherein the shell is a viscoelastic shell.

3. The self-healing shell according to any one of claims 1 to 2, wherein the shell is a capsule or a membrane.

4. The self-healing shell according to any one of claims 1 to 3, wherein the first fluid phase and the second fluid phase form an emulsion; wherein the emulsion may be a water-in-oil emulsion; an oil-in-water emulsion; a water-in-oil-in-water emulsion; an oil-in-water-in-oil emulsion; a triple emulsion; a multiple emulsion; or a double emulsion with multiple cores.

5. The self-healing shell according to any one of claims 1 to 4, wherein the metal-coordinating group is selected from the group consisting of: benzenediol or derivatives thereof, preferably catechol or derivative thereof; benzenetriol or derivatives thereof, preferably gallol or derivatives thereof; histidines and derivatives thereof; groups comprising a carboxyl group; ethylenediaminetetraacetic acid and derivatives thereof; and wherein the metal-coordinating group may optionally be further substituted.

6. The self-healing shell according to any one of claims 1 to 4, wherein the metal-coordinating group is catechol or derivatives thereof.

7. The self-healing shell according to any preceding claim, wherein the metal-coordinating group(s) form a/the hydrophilic group(s).

8. The self-healing shell according to any preceding claim, wherein the one or more hydrophilic group(s) include hydrophilic polymer(s); wherein the hydrophilic polymer(s) may optionally be selected from polyethyleneglycol (PEG), polyacrylicacid (PAA), polyethyleneimine (PEI), polyvinylalcohol (PVA), Poly(N-isopropylacrylamide) (PNIPAM), poly(2-methyl-2-oxazoline) (PMOXA), polyglycols, natural hydrophilic polysaccharides including dextran, alginate, and peptides.

9. The self-healing shell according to any preceding claim, wherein the one or more hydrophobic group(s) include: substituted or unsubstituted lipophilic hydrocarbon chains, fluorinated chains, perfluorinated polyether, hydrophobic polymer(s), polypeptides and/or liquid crystals.

10. The self-healing shell according to claim 9, wherein the one or more hydrophobic group(s) include hydrophobic polymer(s); wherein the hydrophobic polymer(s) may be selected from acrylics, amides and imides, carbonates, dienes, esters, ethers, fluorocarbons, perfluorinated polyethers, olefins, styrenes, vinyl acetals, vinyl and vinylidene chlorides, vinyl esters, vinyl ethers and ketones, vinylpyridine and vinypyrrolidone polymers;
wherein the hydrophobic polymer(s) are more preferably selected from polypropyleneglycol (PPG), polystyrene (PS), polylacticacid (PLA), fluorocarbons, methacrylates, polyethylene, polydimethylsiloxane (PDMS), chitosan and cellulose;
wherein the hydrophobic polymer(s) are particularly preferably selected from fluorocarbons including perfluorinated polyethers, particularly perfluorinated polyethers with carboxylic acid-, methyl ester-, methylene alcohol- or allyl ether end groups. Krytox FSH 157, Krytox FSM 157, Krytox FSL 157, FC40.

11. The self-healing shell according to any preceding claim, wherein the amphiphilic molecules comprise block copolymers;
wherein the block copolymers may be selected from: diblock copolymers, triblock copolymers, and/or random block copolymers;
wherein the diblock copolymers may optionally be selected from: AB diblock copolymers, PEG diblock copolymers, polystyrene diblock copolymers;
wherein the triblock copolymers may optionally be selected from: ABA triblock copolymers, ABC triblock copolymers, biodegradable triblock copolymers, PEG/PPG triblock copolymers, polystyrene triblock copolymers, multi-arm PEG block copolymers, PNIPAM-based block copoylmers, PIMOXA based block-copolymers, light responsive block copolymers, temperature responsive block copolymers Catechol-PPG-Catechol,Catechol-perfluorinated polyether-catechol, perfluorinated polyether-PEG-Catechol, polypeptides; and/or
wherein the random block copolymers may optionally be selected from any combination of the blocks above, provided at least one block is hydrophobic and at least one block is hydrophilic.

12. The self-healing shell according to any preceding claim, wherein the metal cations may be selected from the group consisting of:
metal ions selected from Be²⁺ beryllium ion, Mg²⁺ magnesium ion, Ca²⁺ calcium ion, Sr²⁺ strontium ion, Ba²⁺ barium ion, Ti²⁺ titanium (II), Ti⁴⁺ titanium (IV), Cr²⁺ chromium (II), Cr³⁺ chromium (III), Cr⁶⁺ chromium (VI), Mn²⁺ manganese (II), Mn³⁺ manganese (III), Mn⁴⁺ manganese (IV), Fe²⁺ iron (II), Fe³⁺ iron (III), Co²⁺ cobalt (II), Co³⁺ cobalt (III), Ni²⁺ nickel (II), Ni³⁺ nickel (III), Cu⁺ copper (I), Cu²⁺ copper (II), Ag⁺ silver ion, Au⁺ gold (I), Au⁺³ gold (III), Zn²⁺ zinc ion, Cd²⁺ cadmium ion, Hg₂²⁺ mercury (I), Hg²⁺ mercury (II), Al³⁺ aluminium ion, Ga³⁺ gallium ion, In⁺ indium (I), In³⁺ indium (III), Sn²⁺ tin (II), Sn⁴⁺ tin (IV), Pb²⁺ lead (II), Pb⁴⁺ lead (IV), Bi³⁺ bismuth (III), and/or Bi⁵⁺ bismuth (V); preferably iron, aluminium or titanium; and most preferably iron;
metal oxides, metal carbides, metal nitrides, and/or
metal nanoparticles including iron oxide, iron nitrides, iron carbides, nickel oxides, nickel carbides, titanium oxides, titanium metal particles, titanium nitrides and titanium carbides.

13. A method of manufacturing self-healing shells as claimed in any one of claims 1 to 12; the method comprising forming a system comprising: an interface between a first fluid phase and a second fluid phase, amphiphilic molecules as described in any one of claims 5 to 11 and metal cations as described in claim 12;
wherein the first fluid phase is a liquid phase or a gas phase and the second fluid phase is a liquid phase;
such that the metal-coordinating group(s) and metal cation(s) reversibly cross-link at the interface to form a self-healing shell.

14. The use of a self-healing shell according to any one of claims 1 to 12 in a droplet based screening assay;
wherein the screening assay is preferably high-throughput screening, where the screening can be but is not limited to high throughput drug screening, high throughput antibody screening, or single cell sequencing.

15. An amphiphilic molecule comprising one or more hydrophilic group(s) and one or more hydrophobic group(s); wherein the amphiphilic molecule comprises one or more metal-coordinating group(s); wherein the amphiphilic molecule is a block copolymer; and wherein the metal-coordinating group is a metal-coordinating group selected from the group consisting of: benzenediol or derivatives thereof, preferably catechol or derivatives thereof; and benzenetriol or derivatives thereof, preferably gallol or derivatives thereof; histidines or derivatives thereof; and ethylenediaminetetraacetic acid and derivatives thereof; and wherein the metal-coordinating group(s) may optionally be further substituted.
